**Europäisches Patentamt**

**European Patent Office**

**Office européen des brevets**

(19)

(11) Numéro de publication: **0 167 422**
**B1**

# FASCICULE DE BREVET EUROPÉEN

(12)

(45) Date de publication du fascicule du brevet:
**13.09.89**

(21) Numéro de dépôt: **85400983.4**

(22) Date de dépôt: **20.05.85**

(51) Int. Cl.⁴: **A 61 F 9/02**

(54) **Masque comportant des moyens d'appui sur un visage.**

(30) Priorité: **21.05.84 FR 8407851**

(43) Date de publication de la demande:
**08.01.86 Bulletin 86/2**

(45) Mention de la délivrance du brevet:
**13.09.89 Bulletin 89/37**

(84) Etats contractants désignés:
**AT DE GB IT SE**

(56) Documents cité:
**DE-A-1 193 639**
**FR-A-773 077**
**FR-A-906 927**
**US-A-2 648 843**
**US-A-2 918 676**

(73) Titulaire: **ETABLISSEMENTS BOLLE Georges,**
**Robert et Maurice, 1 et 6 rue Tacon, F-01104**
**Oyonnax (FR)**

(72) Inventeur: **Bolle, Robert, 58 rue Marchon, F-01104**
**Oyonnax (FR)**

(74) Mandataire: **Lepeudry- Gautherat, Thérèse,**
**ARMENGAUD JEUNE CABINET LEPEUDRY 6,**
**rue du Faubourg Saint Honoré, F-75008 Paris (FR)**

## Description

L'invention est relative à un masque, comme par exemple des lunettes de protection ou un masque respiratoire, destiné à être plaqué contre un visage. Elle concerne plus précisément les moyens d'appui du masque sur le visage.

Selon le premier type connu, ces moyens d'appui sont constitués par un cadre de mousse souple qui présente plusieurs inconvénients ce cadre de mousse doit être fixé sur le bord du masque, il est salissant, enfin sa déformabilité est limitée et elle ne permet pas une adaptation correcte à des formes variées de visages.

Selon un second type connu, ces moyens d'appui sont constitués par une lèvre souple en matériau plastique ou élastomère qui s'étend le long du bord du masque et qui est rapportée sur le masque ou d'une seule pièce avec lui.

On connaît également des masques équipés d'une lèvre souple, le visage de l'utilisateur s'appuyant sur cette lèvre souple qui peut fléchir en direction du bord du masque. Mais la possibilité de flexion est réduite car la lèvre souple vient buter contre le bord du masque qui constitue un point dur, ce qui limite la déformabilité de la lèvre et le confort de l'utilisateur. Par ailleurs, cette lèvre s'étend essentiellement d'un côté du bord du masque, c'est-à-dire soit à l'intérieur, soit à l'extérieur du masque, et sa capacité d'amortissement n'est donc pas équilibrée par rapport à des efforts agissant du côté intérieur et du côté extérieur du bord du masque.

Par ailleurs certains masques comportent des pattes de contact mobiles qui peuvent fléchir de façon importante vers l'intérieur du masque. Là encore, l'amortissement s'effectue d'un seul côté du bord du masque vers l'intérieur. En outre, pour une flexion importante de ces pattes de contact mobiles, l'oculaire du masque vient en contact avec le visage de l'utilisateur.

Du document US-A-2 648 843 est connu un masque selon la première partie de la revendication 1.

Aussi un des buts de l'invention est de proposer un masque dont les moyens d'appui sur le visage réagissent de façon sensiblement symétrique à des efforts appliqués du côté extérieur et du côté intérieur du bord du masque.

Un autre but de l'invention est de proposer un masque dont les moyens d'appui peuvent se déformer de façon importante et progressive, tout en maintenant l'oculaire à distance du visage de l'utilisateur.

L'invention concerne à cet effet un masque destiné à être plaqué contre le visage, constitué d'une cage délimitée par une paroi et présentant une ouverture du côté du visage, cette paroi comportant un rebord formé de deux ailes qui s'étendent de part et d'autre du plan de ladite paroi en étant essentiellement non parallèles à cette dernière et en étant déformables alors que la paroi est plutôt rigide, caractérisé en ce que le masque comprend une lèvre souple fixée sur l'une des ailes en une position distante de la paroi, laquelle lèvre s'étend vers le visage, de part et d'autre de la paroi, avec son extrémité libre située en regard mais à distance de l'autre aile et est incurvée en regard du rebord.

De préférence, les ailes sont perpendiculaires à la paroi.

Avantageusement la lèvre est fixée à l'extrémité libre de ladite aile.

De préférence, les ailes et la lèvre sont disposées de façon symétrique par rapport au plan de la paroi.

D'autres détails et avantages de l'invention apparaîtront au cours de la description qui suit, d'un mode de réalisation donné à titre d'exemple non limitatif, en regard des figures annexées, parmi lesquelles:

La figure 1 est une vue en perspective d'un masque de type lunette selon l'invention.

La figure 2 est une vue en coupe transversale suivant la ligne II - II de la figure 1.

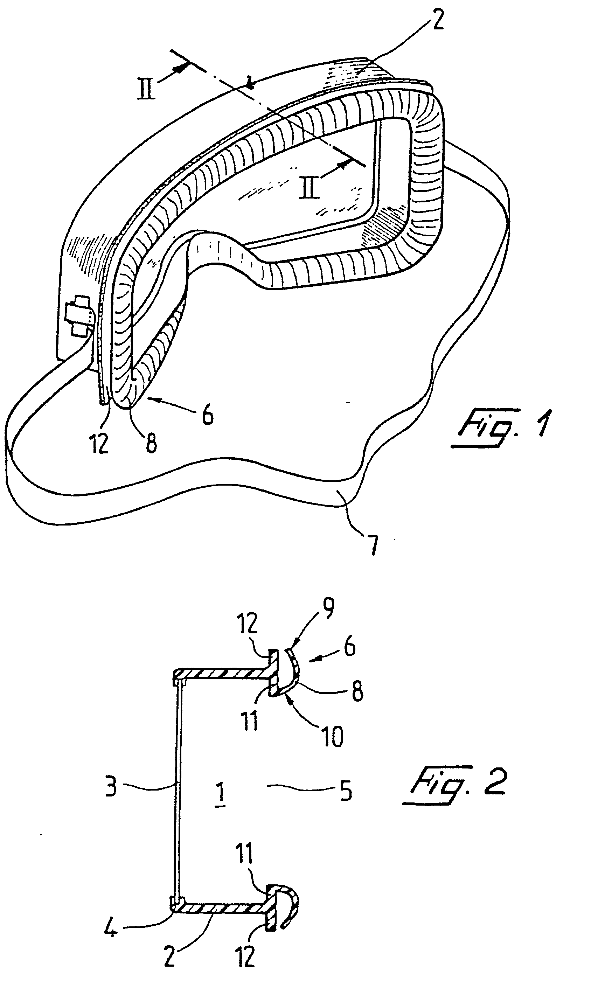

Le masque représenté sur les figures 1 et 2, est destiné à être appliqué devant les yeux pour protéger ceux-ci contre tout agent extérieur. Il est constitué de façon connue en sol d'une cage 1 délimitée par une paroi latérale 2 fermée sur elle-même et par une visière 3 en un matériau transparent s'accrochant périphériquement sur l'un des bords de la paroi latérale 2 en venant pénétrer dans une rainure 4 pratiquée dans ce bord.

La cage ainsi délimitée présente donc une ouverture 5 faisant face à la visière 3. Cette ouverture 5 est bordée par un bourrelet souple 6 par lequel le masque prend appui sur le visage en étant plaqué contre lui par des moyens constitués, dans cet exemple de réalisation, par une sangle élastique 7.

La paroi latérale 2 présente du côté de l'ouverture 5, un rebord périphérique de section en forme de T définissant une aile intérieure 11 s'étendant vers l'intérieur de la cage 1 et une aile extérieure 12 s'étendant à l'extérieur de celle-ci. Les ailes 11, 12 s'étendent donc perpendiculairement au plan de la paroi latérale 2. Ces ailes 11, 12 pourraient également s'étendre obliquement par rapport à la paroi latérale 2, mais toujours de façon essentiellement non parallèle à cette paroi.

Une lèvre souple (8) est disposée en regard desdites ailes 11, 12 et incurvée vers elles. Elle présente un bord intérieur 10 relié à l'extrémité libre de l'aile intérieure 11, et un bord extérieur situé en regard mais à distance de l'extrémité libre de l'aile extérieure 12.

La cage 1 du masque est constituée de façon connue en soi en une matière thermoplastique ou élastomère moulée d'une seule pièce, la visière thermoplastique 3 étant indépendante et rapportée sur la paroi latérale 2. La rigidité nécessaire de la cage 1 est obtenue en donnant une épaisseur suffisante à la visière 3 et à la paroi 2. La souplesse des ailes 11, 12 et de la lèvre du rebord est obtenue par une épaisseur inférieure à celle de la paroi 2.

Dans la pratique, lorsque le masque s'appuie sur le visage d'un utilisateur, les moyens d'appui constitués par la lèvre et les ailes 11, 12 du rebord de la paroi 2 se déforment en deux temps.

Dans un premier temps, seule la lèvre 8 fléchit en direction des ailes 11, 12. Dans un second temps, et pour un effort plus important, la lèvre 8 s'appuie sur l'une et/ou l'autre des ailes 11, 12 qui fléchissent à leur tour en direction de la paroi 2. Par exemple, pour un effort s'appliquant du côté du bord extérieur 9 de la lèvre 8, celle-ci vient au contact de l'aile 12 qui fléchit alors. De façon sensiblement symétrique, pour un effort s'appliquant du côté du bord intérieur 10 de la lèvre 8, celle-ci vient au contact de l'aile 11 qui fléchit. Quant à la paroi 2 du masque, sa rigidité est suffisante pour qu'elle ne fléchisse quasiment pas. Elle peut même être conçue de façon totalement rigide.

On notera donc que les moyens d'appui selon l'invention réagissent de façon symétrique et progressive à des efforts intérieurs et extérieurs au masque. Ils offrent une grande capacité de déformation, et donc d'amortissement: à la différence des masques connus, le lèvre 8 une fois déformée ne bute pas contre un rebord indéformable, mais elle provoque au contraire la déformation du rebord. Enfin, le rebord 11, 12 est toujours maintenu à distance de la visière 3 du masque, grâce à la paroi 2 qui est suffisamment rigide et disposée perpendiculairement au visage de l'utilisateur.

Des variantes peuvent être apportées au mode de réalisation décrit ci-dessus on peut notamment relier la lèvre 8 à la paroi 2 en la fixant par son bord extérieur 9 à l'extrémité libre de l'aile extérieure 12, de façon à constituer un bourrelet ouvert vers l'intérieur de la cage 1.

## Revendications

1. Masque destiné à être plaqué contre le visage, constitué d'une cage délimitée par une paroi et présentant une ouverture du côté du visage, ladite paroi (2) comportant un rebord formé de deux ailes (11, 12) qui s'étendent de part et d'autre du plan de ladite paroi (2) en étant essentiellement non parallèles à cette dernière et en étant déformables alors que la paroi est plutôt rigide, caractérisé en ce que le masque comprend une lèvre souple (8) fixée sur l'une des ailes (11, 12) en une position distante de la paroi (2), laquelle lèvre s'étend vers le visage de part et d'autre de ladite paroi (2) avec son extrémité libre située en regard mais à distance de l'autre aile et est incurvée en regard dudit rebord.

2. Masque selon la revendication 1, caractérisé en ce que lesdites ailes (11, 12) sont perpendiculaires à ladite paroi (2).

3. Masque selon la revendication 1 ou la revendication 2, caractérisé en ce que ladite lèvre (8) est fixée à l'extrémité libre de ladite aile (11).

4. Masque selon l'une quelconque des revendications 1 à 3, caractérisé en ce que lesdites ailes (11, 12) et ladite lèvre (8) sont disposées de façon symétrique par rapport au plan de ladite paroi (2).

## Patentansprüche

1. Maske, die zur Anlage an einem Gesicht bestimmt ist, mit einem Käfig, der von einer Wand begrenzt ist, die eine Öffnung zum Gesicht aufweist, wobei die Wand (2) eine von zwei Flügeln (11, 12) gebildete Randleiste hat, welche sich zu beiden Seiten der Ebene dieser Wand (2) erstrecken, in hohem Maße nicht parallel zu dieser verlaufen und deformierbar sind, wohingegen die Wand im wesentlichen steif ist, dadurch gekennzeichnet, daß die Maske eine nachgiebige Lippe (8) aufweist, die an einem der Flügel (11, 12) in einer von der Wand (2) entfernten Position befestigt ist, die sich beidseits der Wand (2) zum Gesicht hin erstreckt, wobei ihr freies Ende in Abstand dem anderen Flügel gegenüberliegt und die gegenüber der Randleiste gekrümmt ist.

2. Maske nach Anspruch 1, dadurch gekennzeichnet, daß die Flügel (11, 12) rechtwinklig zur Wand (2) verlaufen.

3. Maske nach Anspruch 1 oder 2, dadurch gekennzeichnet, daß die Lippe (8) am freien Ende des genannten Flügels (11) befestigt ist.

4. Maske nach einem der Ansprüche 1 bis 3, dadurch gekennzeichnet, daß die Flügel (11, 12) und die Lippe (8) symmetrisch zur Ebene der Wand (2) angeordnet sind.

## Claims

1. Mask which is to be placed against the face and is formed by a cage delimited by a wall and having an opening on the side towards the face, the said wall (2) comprising a rim formed by two wings (11, 12) which extend one on each side of the plane of the said wall (2) being substantially non-parallel thereto and being deformable while the wall is fairly rigid, characterised in that the mask comprises a flexible lip (8) which is fastened to one of the wings (11, 12) in a position spaced away from the wall (2) which lip extends towards the face, on each side of the said wall (2), with its free end facing, but at a distance from, the other wing, and is concave towards the said rim.

2. Mask according to claim 1, characterised in that the said wings (11, 12) are perpendicular to the said wall (2).

3. Mask according to claim 1 or claim 2, characterised in that the said lip (8) is fastened to the free end of the said wing (11).

4. Mask according to any one of claims 1 to 3, characterised in that the said wings (11, 12) and the said lip (8) are arranged symmetrically with respect to the plane of the said wall (2).

*Fig. 1*

*Fig. 2*